# EUROPEAN PATENT APPLICATION

(11) **EP 3 147 658 A1**
(43) Date of publication of application: **29.03.2017**
(21) Application number: 16794913.0
(22) Date of filing: 24.03.2016
(51) Int. Cl.: G01N 27/404, A61B 1/12, A61L 2/18, G01N 27/26, G01N 27/333, G01N 27/416

(54) **ENDOSCOPE REPROCESSOR**

(30) Priority: 21.07.2015 JP 2015144145
(71) Applicant: OLYMPUS CORPORATION, Shibuya-ku Tokyo 151-0072 (JP)
(72) Inventor: IWASAKI, Tomokazu, Tokyo 151-0072 (JP)
(74) Representative: Schicker, Silvia
(86) International application number: PCT/JP2016/059502
(87) International publication number: WO 2017/013906

(57) **Abstract**

An endoscope reprocessor invention includes: a medicinal solution tank configured to store medicinal solution; a concentration measuring portion arranged in the medicinal solution tank so that a permeation membrane is submerged in the medicinal solution; a gas generating portion including a vaporizing portion configured to vaporize a part of the medicinal solution to generate gas and arranged at a position where the gas that floats upward in the medicinal solution is in contact with the permeation membrane; and a controlling portion connected to the concentration measuring portion and the gas generating portion and configured to perform first control to drive the concentration measuring portion in a state that the gas generating portion is stopped and second control to drive the concentration measuring portion in a state that the gas generating portion is driven.

## Description

### Technical Field

The present invention relates to an endoscope reprocessor provided with a concentration meter using a permeation membrane.

### Background Art

For an endoscope used in a medical field, after using the endoscope, reprocessing using medicinal solution, such as cleaning treatment and disinfecting treatment, is performed. Further, an endoscope reprocessor configured to automatically perform the endoscope reprocessing is known. For example, Japanese Patent Laid-Open Publication No. 2010-57792 discloses an endoscope reprocessor provided with a concentration measuring portion configured to measure a concentration of medicinal solution used for the reprocessing.

As the concentration measuring portion, such that is in a form of using a permeation membrane which causes particular ions in medicinal solution to permeate the permeation membrane is known. In a case of measuring a concentration of medicinal solution using the concentration measuring portion of the form, a measurement surface, which is a part where the permeation membrane is provided, is caused to be in contact with the medicinal solution.

When the measurement surface to be in contact with the medicinal solution is in a dry state at the time of concentration measurement, the concentration measuring portion using the permeation membrane requires a longer standby time period until a correct measurement result is obtained after the measurement surface comes into contact with the medicinal solution, in comparison with a case where the measurement surface is in a wet state.

The present invention solves the problem described above, and an object is to provide an endoscope reprocessor capable of executing concentration measurement without delay even if the permeation membrane of the concentration measuring portion is in a dry state.

### Disclosure of Invention

### Means for Solving the Problem

An endoscope reprocessor of an aspect of the present invention includes: a medicinal solution tank configured to store medicinal solution; a concentration measuring portion arranged in the medicinal solution tank so that a permeation membrane is submerged in the medicinal solution; a gas generating portion including a vaporizing portion configured to vaporize a part of the medicinal solution to generate gas and arranged at a position where the gas that floats upward in the medicinal solution is in contact with the permeation membrane; and a controlling portion connected to the concentration measuring portion and the gas generating portion and configured to perform first control to drive the concentration measuring portion in a state that the gas generating portion is stopped and second control to drive the concentration measuring portion in a state that the gas generating portion is driven.

### Brief Description of the Drawings

Fig. 1 is a diagram showing a configuration of an endoscope reprocessor of a first embodiment;
Fig. 2 is a diagram showing a configuration of a medicinal solution tank, a concentration measuring portion and a gas generating portion of the first embodiment;
Fig. 3 is a diagram schematically showing a section of a permeation membrane;
Fig. 4 is a flowchart of a concentration measurement operation of the endoscope reprocessor of the first embodiment;
Fig. 5 is a diagram showing a state that the gas generating portion has been driven in the first embodiment;
Fig. 6 is a diagram showing a modification of a vaporizing portion of the first embodiment;
Fig. 7 is a diagram showing a configuration of an endoscope reprocessor of a second embodiment;
Fig. 8 is a flowchart showing an operation of a concentration judging portion of the second embodiment;
Fig. 9 is a diagram showing a configuration of an endoscope reprocessor of a third embodiment;
Fig. 10 is a diagram showing a modification of a removing portion of the third embodiment;
Fig. 11 is a diagram showing a configuration of an endoscope reprocessor of a fourth embodiment; and
Fig. 12 is a diagram showing a configuration of an endoscope reprocessor of a fifth embodiment.

### Best Mode for Carrying Out the Invention

Preferred embodiments of the present invention will be described below with reference to drawings. Note that, in each drawing used in the description below, a reduced scale differs for each component so that the component is of a recognizable size on the drawing. The present invention is not limited only to the number of components, shapes of the components, a ratio of sizes of the components, or a relative positional relationship among the respective components shown in the drawings.

### (First Embodiment)

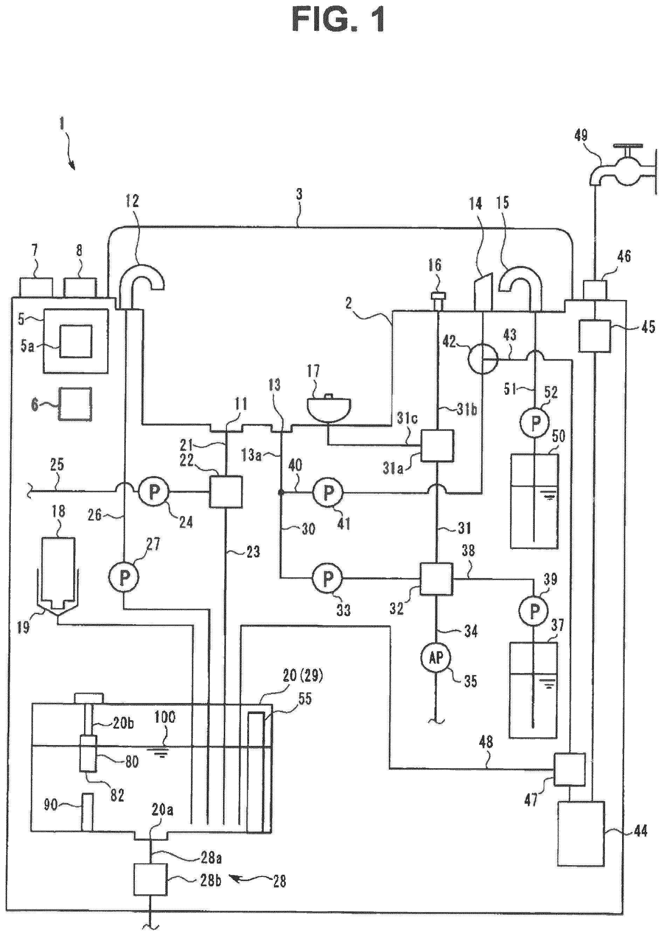

An example of an embodiment of the present invention will be described below. An endoscope reprocessor 1 shown in Fig. 1 is an apparatus configured to perform reprocessing for an endoscope. The reprocessing stated here is not especially limited. Any of rinsing treatment with water, cleaning treatment for washing out dirt such as organic matters, disinfecting treatment for inactivating predetermined microorganisms, sterilizing treatment for removing or extinguishing all microorganisms and a combination of those may be adopted.

Note that, in the description below, "upward" refers to a position farther away from ground than a comparison target, and "downward" refers to a position closer to the ground than the comparison target. Further, "height" in the description below indicates a height relationship along a gravity direction.

The endoscope reprocessor 1 is provided with a controlling portion 5, a power source portion 6, a treatment tank 2, a medicinal solution tank 20, a concentration measuring portion 80 and a gas generating portion 90.

The controlling portion 5 can be configured, being provided with an operation device (CPU), a storage device (RAM), an auxiliary storage device, an input/output device, a power control device and the like, and has a configuration for controlling an operation of each of the portions constituting the endoscope reprocessor 1 based on a predetermined program. The controlling portion 5 includes a judging portion 5a configured to execute a judgement process to be described later. An operation of each component included in the endoscope reprocessor 1 in the description below is controlled by the controlling portion 5 even when it is not especially described.

The power source portion 6 supplies power to each portion of the endoscope reprocessor 1. The power source portion 6 distributes power obtained from outside, such as from a commercial power supply, to each portion. Note that the power source portion 6 may be provided with a power generating device or a battery.

The treatment tank 2 is in a concave shape having an opening section, and is capable of storing liquid inside. An endoscope not shown can be arranged inside the treatment tank 2. In the present embodiment, a cover 3 configured to open/close the opening section of the treatment tank 2 is provided on a top of the treatment tank 2, as an example. In a case of performing reprocessing for an endoscope inside the treatment tank 2, the opening section of the treatment tank 2 is closed with the cover 3.

The treatment tank 2 is provided with a medicinal solution nozzle 12, a liquid discharge port 11, a circulation port 13, a circulation nozzle 14, a cleaning liquid nozzle 15, an endoscope connecting portion 16 and an accessory case 17.

The medicinal solution nozzle 12 is an opening section communicating with the medicinal solution tank 20 via a medicinal solution conduit 26. The medicinal solution tank 20 stores medicinal solution.

The medicinal solution conduit 26 is provided with a medicinal solution pump 27. By operating the medicinal solution pump 27, the medicinal solution in the medicinal solution tank 20 is transferred into the treatment tank 2 via the medicinal solution conduit 26 and the medicinal solution nozzle 12.

In the medicinal solution tank 20, the concentration measuring portion 80 configured to measure a concentration of medicinal solution 100 is retained by a retaining portion 20b. The concentration measuring portion 80 may be fixed to the retaining portion 20b or may be attachably and detachably retained by the retaining portion 20b. Further, a water level sensor 55 and the gas generating portion 90 are arranged in the medicinal solution tank 20. The concentration measuring portion 80, the water level sensor 55 and the gas generating portion 90 will be described later.

A kind of the medicinal solution stored in the medicinal solution tank 20 is not especially limited. In the present embodiment, the medicinal solution is, for example, disinfectant liquid used for disinfecting treatment such as peracetic acid. The present invention, however, is not limited to disinfectant liquid. Cleaning liquid used for cleaning treatment, highly volatile solution used for drying or the like may be appropriately selected depending on purposes.

Further, in the present embodiment, the medicinal solution is such that is obtained by diluting stock solution of the medicinal solution supplied from a medicinal solution bottle 18 with water at a predetermined rate, as an example. The medicinal solution tank 20 of the present embodiment communicates with a bottle connecting portion 19 configured to introduce the stock solution of the medicinal solution supplied from the medicinal solution bottle 18 into the medicinal solution tank 20 and a dilution conduit 48 configured to introduce the water for dilution into the medicinal solution tank 20. By the medicinal solution bottle 18 being connected to the bottle connecting portion 19, the stock solution of the medicinal solution is introduced into the medicinal solution tank 20. A configuration for introducing the water into the medicinal solution tank 20 from the dilution conduit 48 will be described later.

Note that the endoscope reprocessor 1 may not have the configuration for diluting medicinal solution with water or the like. Further, in a case of medicinal solution used by mixing a plurality of kinds of stock solutions, the bottle connecting portion 19 can be connected to a plurality of medicinal solution bottles 18.

Further, in the present embodiment, when a concentration of medicinal solution is within a predetermined efficacious range, the medicinal solution is reusable, as an example. The medicinal solution tank 20 also serves as a medicinal-solution collecting section 29 configured to collect medicinal solution transferred into the treatment tank 2 from inside the medicinal solution tank 20 and store the medicinal solution again. In description below, when the medicinal solution tank 20 and the medicinal-solution collecting section 29 are not distinguished from each other, the medicinal solution tank 20/ medicinal-solution collecting section 29 will be merely referred to as the medicinal solution tank 20.

Note that the medicinal solution tank 20 may be provided separately from the medicinal-solution collecting section 29. In the case where the medicinal solution tank 20 is configured separately from the medicinal-solution collecting section 29, capacity of the medicinal solution tank 20 may be smaller than that of the medicinal-solution collecting section 29.

Further, a liquid discharge section 28 is arranged on the medicinal solution tank 20. The liquid discharge section 28 discharges liquid such as medicinal solution and water from inside the medicinal solution tank 20. The liquid discharge section 28 may be configured to discharge the liquid from inside the medicinal solution tank 20 by gravity or may be configured to forcibly discharge the liquid from inside the medicinal solution tank 20 by a pump.

In the present embodiment, the liquid discharge section 28 includes a drain conduit 28a communicating with a liquid discharge port 20a provided on or near a bottom surface of the medicinal solution tank 20, and a drain valve 28b configured to open/close the drain conduit 28a, as an example. The drain valve 28b may be a solenoid valve which is opening/closing-controlled by the controlling portion 5 or may be a cock which is opened/closed by a user's manual operation.

Note that a route for discharging liquid from inside the medicinal solution tank 20 is not limited to a drain conduit. For example, by starting operation of the medicinal solution pump 27, the liquid can be discharged from inside the medicinal solution tank 20 into the treatment tank 2 via the medicinal solution conduit 26 and the medicinal solution nozzle 12.

The liquid discharge port 11 is an opening section provided at a lowest position in the treatment tank 2. The liquid discharge port 11 is connected to a discharge conduit 21. The discharge conduit 21 causes the liquid discharge port 11 and a switching valve 22 to communicate with each other. A collection conduit 23 and a discarding conduit 25 are connected to the switching valve 22. The switching valve 22 can switch among a state that the discharge conduit 21 is blocked, a state that the discharge conduit 21 and the collection conduit 23 communicate with each other, and a state that the discharge conduit 21 and the discarding conduit 25 communicate with each other.

The collection conduit 23 causes the medicinal solution tank 20 and the switching valve 22 to communicate with each other. Further, the discarding conduit 25 is provided with a discharge pump 24. The discarding conduit 25 is connected to a liquid discharge facility for receiving liquid discharged from the endoscope reprocessor 1.

By causing the switching valve 22 to be in the closed state, liquid can be stored in the treatment tank 2. Further, by causing the switching valve 22 to be in the state that the discharge conduit 21 and the collection conduit 23 communicate with each other when medicinal solution is stored in the treatment tank 2, the medicinal solution is transferred from the treatment tank 2 to the medicinal solution tank 20. Further, by causing the switching valve 22 to be in the state that the discharge conduit 21 and the discarding conduit 25 communicate with each other and starting operation of the discharge pump 24, liquid in the treatment tank 2 is sent out to the liquid discharge facility via the discarding conduit 25.

The circulation port 13 is an opening section provided near the bottom surface of the treatment tank 2. The circulation port 13 communicates with a circulation conduit 13a. The circulation conduit 13a branches to two conduits of an endoscope circulation conduit 30 and a treatment tank circulation conduit 40.

The endoscope circulation conduit 30 causes the circulation conduit 13a and a channel valve 32 to be described later to communicate with each other. The endoscope circulation conduit 30 is provided with a circulation pump 33. The circulation pump 33 transfers fluid in the endoscope circulation conduit 30 toward the channel valve 32 by operating.

In addition to the endoscope circulation conduit 30 described before, an air feeding conduit 34, an alcohol conduit 38 and a delivery conduit 31 are connected to the channel valve 32. The channel valve 32 selectively causes any one of the endoscope circulation conduit 30, the air feeding conduit 34 and the alcohol conduit 38 to communicate with the delivery conduit 31.

One end portion of the air feeding conduit 34 is left open in air, and the other end portion is connected to the channel valve 32. Note that one end portion of the air feeding conduit 34 is provided with a filter for filtering passing air though it is not shown. An air pump 35 is provided in the air feeding conduit 34 and transfers gas in the air feeding conduit 34 toward the channel valve 32 by operating.

The alcohol conduit 38 causes an alcohol tank 37 configured to store alcohol and the channel valve 32 to communicate with each other. The alcohol stored in the alcohol tank 37 is, for example, ethanol. A concentration of the alcohol can be appropriately selected. An alcohol pump 39 is provided on the alcohol conduit 38 and transfers the alcohol in the alcohol tank 37 toward the channel valve 32 by operating.

By, when liquid is stored in the treatment tank 2, causing the channel valve 32 to be in a state that the delivery conduit 31 and the endoscope circulation conduit 30 communicate with each other and starting operation of the circulation pump 33, the liquid in the treatment tank 2 is sent into the delivery conduit 31 via the circulation port 13, the circulation conduit 13a and the endoscope circulation conduit 30.

Further, by causing the channel valve 32 to be in a state that the delivery conduit 31 and the air feeding conduit 34 communicate with each other and starting operation of the air pump 35, air is sent into the delivery conduit 31. Further, by causing the channel valve 32 to be in a state that the delivery conduit 31 and the alcohol conduit 38 communicate with each other and starting operation of the alcohol pump 39, alcohol in the alcohol tank 37 is sent into the delivery conduit 31.

The delivery conduit 31 branches to an endoscope connection conduit 31b and a case connection conduit 31c. The endoscope connection conduit 31b is connected to the endoscope connecting portion 16. Further, the case connection conduit 31 is connected to the accessory case 17.

Further, the delivery conduit 31 is provided with a flow path switching portion 31 a. The flow path switching portion 31 a can switch whether fluid sent into the delivery conduit 31 from the channel valve 32 is to flow into the endoscope connection conduit 31b or into the case connection conduit 31c. Note that, at time of switching, control may be performed so that pressure on the endoscope connection conduit 31b side is constant.

The endoscope connecting portion 16 is connected to a pipe sleeve provided on an endoscope via an endoscope tube not shown. Further, the accessory case 17 is a basket-shaped member configured to contain accessories of an endoscope, which are not shown. Therefore, the fluid sent into the delivery conduit 31 from the channel valve 32 is introduced into the pipe sleeve of an endoscope or the accessory case 17.

The treatment tank circulation conduit 40 causes the circulation conduit 13a and the circulation nozzle 14 to communicate with each other. The circulation nozzle 14 is an opening section provided on the treatment tank 2. A liquid flow generating pump 41 is provided in the treatment tank circulation conduit 40.

Further, a three-way valve 42 is provided between the liquid flow generating pump 41 of the treatment tank circulation conduit 40 and the circulation nozzle 14. A water supply conduit 43 is connected to the three-way valve 42. The three-way valve 42 can switch between a state that the circulation nozzle 14 and the treatment tank circulation conduit 40 communicate with each other and a state that the circulation nozzle 14 and the water supply conduit 43 communicate with each other.

The water supply conduit 43 causes the three-way valve 42 and a water supply source connecting portion 46 to communicate with each other. The water supply conduit 43 is provided with a water introducing valve 45 configured to open/close the water supply conduit 43 and a water filter 44 configured to filter water. The water supply source connecting portion 46 is connected to a water supply source 49 configured to send out water, such as water utility, for example, via a hose.

A dilution valve 47 is provided in a section between the water filter 44 and the three-way valve 42 in the water supply conduit 43. The dilution conduit 48 causing the dilution valve 47 and the medicinal solution tank 20 to communicate to each other is connected to the dilution valve 47. The dilution valve 47 can switch between a state that the water filter 44 and the three-way valve 42 communicate with each other and a state that the water filter 44 and the dilution conduit 48 communicate with each other.

By, when liquid is stored in the treatment tank 2, causing the three-way valve 42 to be in the state that the circulation nozzle 14 and the treatment tank circulation conduit 40 communicate with each other, and causing the dilution valve 47 to be in the state that the water filter 44 and the three-way valve 42 communicate with each other, and starting operation of the liquid flow generating pump 41, the liquid in the treatment tank 2 is ejected from the circulation nozzle 14 via the circulation port 13, the circulation conduit 13a and the treatment tank circulation conduit 40.

Further, by causing the three-way valve 42 to be in the state that the circulation nozzle 14 and the water supply conduit 43 communicate with each other, causing the dilution valve 47 in the state that the water filter 44 and the three-way valve 42 communicate with each other, and causing the water introducing valve 45 in an open state, water supplied from the water supply source 49 is ejected from the circulation nozzle 14. The liquid ejected from the circulation nozzle 14 is introduced into the treatment tank 2.

Further, by causing the dilution valve 47 to be in the state that the water filter 44 and the dilution conduit 48 communicate with each other and causing the water introducing valve 45 to be in the open state, the water supplied from the water supply source 49 is introduced into the medicinal solution tank 20.

The cleaning liquid nozzle 15 is an opening section for communicating to a cleaning liquid tank 50 storing cleaning liquid, via a cleaning liquid conduit 51. The cleaning liquid is used for cleaning treatment. The cleaning liquid conduit 51 is provided with a cleaning liquid pump 52. By operating the cleaning liquid pump 52, the cleaning liquid in the cleaning liquid tank 50 is transferred into the treatment tank 2.

Further, the endoscope reprocessor 1 is provided with an operation portion 7 and an output portion 8 constituting a user interface configured to give and receive information to and from a user. The operation portion 7 and the output portion 8 are electrically connected to the controlling portion 5.

The operation portion 7 includes operation members such as a push switch and a touch sensor. Further, the output portion 8 includes, for example, a display device configured to display an image and characters, a light emitting device configured to emit light, a speaker configured to emit a sound, or a combination of those. Note that the operation portion 7 and the output portion 8 may be in a form of being provided on an electronic apparatus configured to perform wireless communication with the controlling portion 5.

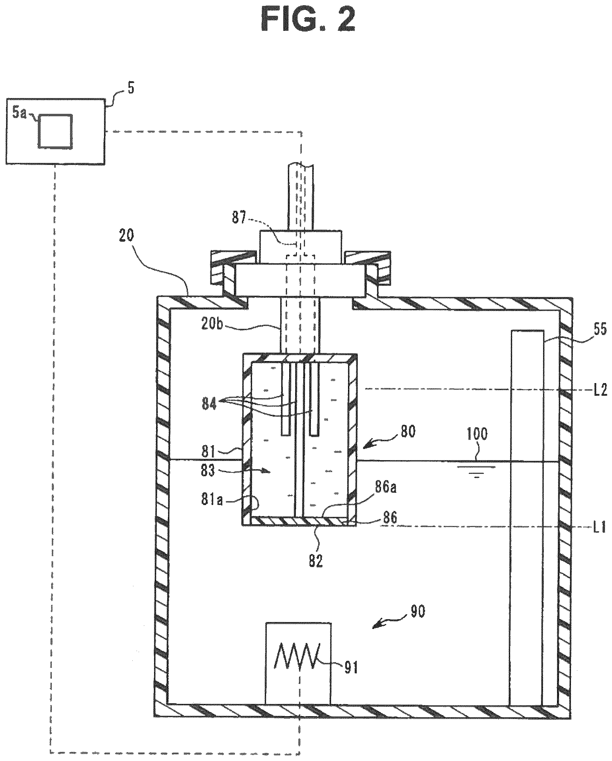

Next, a configuration of the medicinal solution tank 20 will be described. As shown in Fig. 2, the concentration measuring portion 80, the water level sensor 55 and the gas generating portion 90 are arranged in the medicinal solution tank 20.

The medicinal solution tank 20 can store medicinal solution up to a second water level L2 higher than a predetermined first water level L1. The medicinal solution tank 20 has the retaining portion 20b configured to retain the concentration measuring portion 80, which is described later.

The concentration measuring portion 80 measures a concentration of a measurement target in liquid or gas which is in contact with a measurement surface 82. In the present embodiment, the concentration measuring portion 80 is electrically connected to the controlling portion 5, and information about a measurement result of a medicinal solution concentration measured by the concentration measuring portion 80 is inputted to the controlling portion 5, as an example.

The concentration measuring portion 80 includes a housing 81, electrodes 84, a permeation membrane 86 and internal liquid 83. The housing 81 is a container-shaped member on which a recess 81 a is provided.

The plurality of electrodes 84 are arranged inside the recess 81a, being separated from one another. The plurality of electrodes 84 are connected to a control device of the concentration measuring portion 80, which is not shown, via an electrical cable 87. Note that the control device of the concentration measuring portion 80 may be configured integrally with the housing 81. As described before, the control device of the concentration measuring portion 80 is included in the controlling portion 5 in the present embodiment.

An opening section of the recess 81a is covered with the permeation membrane 86. Further, the internal liquid 83 is stored inside the recess 81a. An internal face 86a of the permeation membrane 86, which is exposed on an inner side of the recess 81a is in contact with the internal liquid 83. Further, the plurality of electrodes 84 are immersed in the internal liquid 83 inside the recess 81a.

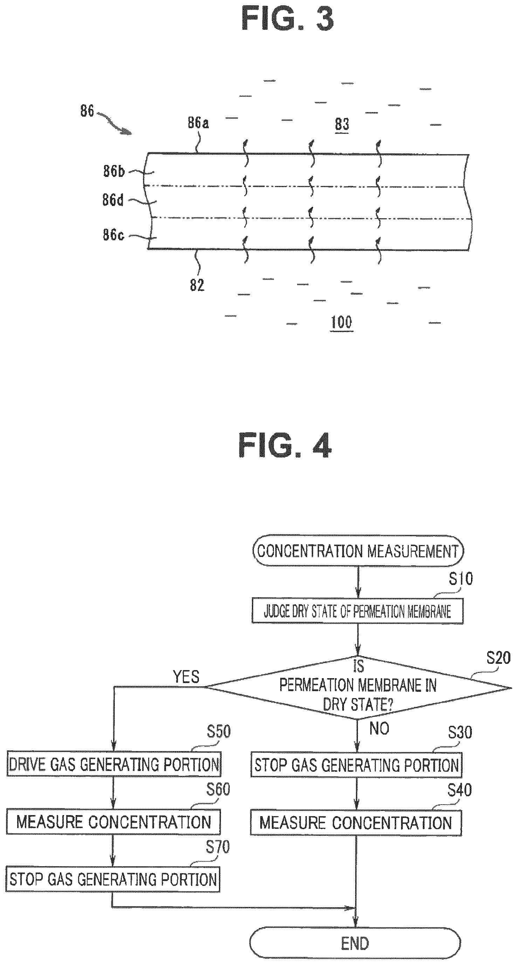

The measurement surface 82 of the concentration measuring portion 80 is a face on an opposite side of the internal face 86a in the permeation membrane 86. Fig. 3 is a diagram schematically showing that matter permeates the permeation membrane 86. Fig. 3 shows a case where the medicinal solution 100 is in contact with the measurement surface 82, and the measurement surface 82 side of the permeation membrane 86 is in a wet state. Further, in the state shown in Fig. 3, a concentration of measurement target matter in the medicinal solution 100 is higher than a concentration of the measurement target matter in the internal liquid 83.

The permeation membrane 86 is a porous membrane which does not allow liquid molecules to pass but allows gas molecules to pass through. On a section of the permeation membrane 86 which is in a state of being arranged so as to separate the internal liquid 83 and the medicinal solution 100 from each other, a first area 86b which the internal liquid 83 has permeated, a second area 86c which the medicinal solution 100 has permeated, and a dry area 86d which is dry between the first area 86b and the second area 86c exist. Gas of the medicinal solution 100 which has evaporated in the second area 86c of the permeation membrane 86 permeates the dry area 86d and is dissolved in the internal liquid 83 in the first area 86b.

Since the internal liquid 83 is always stored in the recess 81 a, the internal liquid 83 continues to be in contact with the internal face 86a of the permeation membrane 86, and a thickness of the first area 86b is always almost constant. The thickness of the first area 86b is a depth of permeation of the internal liquid 83 into the permeation membrane 86 from the internal face 86a. On the other hand, since the medicinal solution 100 may be discharged from inside the medicinal solution tank 20, the measurement surface 82 of the permeation membrane 86 is not always in contact with liquid. Therefore, if the measurement surface 82 continues to be exposed in air, an amount of moisture of the second area 86c gradually decreases. Then, if the measurement surface 82 continues to be exposed in air, the second area 86c disappears in the end, and the dry area 86d reaches the measurement surface 82.

If the measurement surface 82 is in a wet state, and liquid is in contact with the measurement surface 82, then the concentration of the measurement target matter in the internal liquid 83 changes according to a concentration of the measurement target matter in the liquid in contact with the measurement surface 82.

Further, if the measurement surface 82 is in a dry state, and gas is in contact with the measurement surface 82, then the concentration of the measurement target matter in the internal liquid 83 changes according to a concentration of a measurement target component in the gas in contact with the measurement surface. Here, a measurement target component in gas is measurement target matter in liquid.

As described above, inside the recess 81 a of the housing 81 of the concentration measuring portion 80, the internal liquid 83 is arranged between the electrodes 84 and the permeation membrane 86, and the electrodes 84 and the permeation membrane 86 are in a state of being connected to each other via the internal liquid 83. The state of "being connected to each other" stated here refers to a state that the measurement target matter which has permeated the permeation membrane 86 and reached an inside of the internal liquid 83 can reach the electrodes 84, with the internal liquid 83 as a medium.

According to the concentration of the measurement target matter in the internal liquid 83, a change occurs in potential differences caused among the plurality of electrodes 84 or a value of a current which flows between paired electrodes 84. The concentration measuring portion 80 measures the change in the potential differences caused among the plurality of electrodes 84 or the change in the value of the current which flows between the paired electrodes 84 and, based on a value of the measurement, measures the concentration of the measurement target matter in liquid or gas in contact with the measurement surface 82. Since a principle and configuration of such concentration measurement by the concentration measuring portion 80 is well known, detailed description will be omitted.

The retaining portion 20b which the medicinal solution tank 20 has retains the concentration measuring portion 80 so that the measurement surface 82 of the permeation membrane 86 of the concentration measuring portion 80 is in contact with the medicinal solution 100, inside the medicinal solution tank 20. The concentration measuring portion 80 is retained by the retaining portion 20b at a predetermined position relative to the medicinal solution tank 20.

More specifically, the measurement surface 82 of the concentration measuring portion 80 retained by the retaining portion 20b is arranged at the predetermined first water level L1 in the medicinal solution tank 20. In the present embodiment, the measurement surface 82 faces downward in the state that the concentration measuring portion 80 is retained by the retaining portion 20b, as an example. Therefore, if the medicinal solution 100 is stored to the predetermined first water level L1 of the medicinal solution tank 20 or higher, the measurement surface 82 is submerged in the medicinal solution 100. Note that a part of the concentration measuring portion 80 in the state of being retained by the retaining portion 20b may be exposed outside the medicinal solution tank 20.

The water level sensor 55 detects a height of a surface of liquid stored in the medicinal solution tank 20. The water level sensor 55 is electrically connected to the controlling portion 5 and outputs information about a detection result to the controlling portion 5. In the present embodiment, the water level sensor 55 detects at least whether the liquid surface in the medicinal solution tank 20 has reached the first water level L1 or not, as an example.

Note that the water level sensor 55 may be used to, in a case of mixing the stock solution of the medicinal solution supplied from the medicinal solution bottle 18 and water supplied from the dilution conduit in the medicinal solution tank 20, cause a volume ratio of both to be a predetermined value.

The configuration of the water level sensor 55 is not especially limited. The water level sensor 55 may be, for example, a so-called electrode-type water level sensor which is provided with a plurality of electrodes arranged being separated from one another and configured to detect whether a liquid surface has reached a predetermined water level or not based on whether there is electrical continuity among the plurality of electrodes which changes according to whether the plurality of electrodes are submerged in liquid or not. Further, for example, the water level sensor 55 may be a so-called float-type water level sensor configured to detect whether a liquid surface of medicinal solution has reached a predetermined water level based on an operation state of a switch configured to open or close in response to a vertical motion of a float floating in the medicinal solution.

The gas generating portion 90 is provided with a vaporizing portion 91 configured to vaporize a part of the medicinal solution 100 in the medicinal solution tank 20 to generate gas. The gas generating portion 90 is connected to the controlling portion 5, and an operation of the gas generating portion 90 is controlled by the controlling portion 5.

A configuration of the vaporizing portion 91 is not especially limited if it is possible to vaporize the medicinal solution 100. In the present embodiment, the vaporizing portion 91 is provided with a heater configured to heat the medicinal solution 100 up to a boiling point, as an example. By the vaporizing portion 91, which is the heater, being driven, the medicinal solution 100 around the vaporizing portion 91 evaporates.

Note that the heater constituting the vaporizing portion 91 may be also configured as a heat retaining portion configured to retain a temperature of the medicinal solution 100 stored in the medicinal solution storing portion 20 at a predetermined temperature. In this case, the endoscope reprocessor 1 is provided with a temperature measuring portion configured to measure the temperature of the medicinal solution 100 in the medicinal solution tank 20 and an adjusting portion configured to adjust output of the heater. Further, in this case, the controlling portion 5 is connected to the temperature measuring portion and the adjusting portion, and retains the temperature of the medicinal solution 100 stored in the medicinal solution storing portion 20 at the predetermined temperature by controlling the adjusting portion in accordance with a measurement result of the temperature measuring portion.

Note that the configuration of the vaporizing portion 91 is not limited to that of the present embodiment. For example, the vaporizing portion 91 may be provided with a vacuum pump or the like and configured to vaporize the medicinal solution 100 by causing pressure of the medicinal solution 100 to be lower than saturated vapor pressure. Further, for example, the vaporizing portion 91 may be configured to vaporize the medicinal solution 100 by causing the pressure of the medicinal solution 100 to be lower than the saturated vapor pressure by driving an ultrasound transducer or rotating a propeller. Note that the gas generating portion 90 may be provided with a water level sensor for preventing the vaporizing portion 91 from being driven if the vaporizing portion 91 is exposed in air in the medicinal solution tank 20.

The gas generating portion 90 releases gas generated by the vaporizing portion 91 being driven, into the medicinal solution 100. If the medicinal solution 100 is stored to the predetermined first water level L1 or higher in the medicinal solution tank 20, the gas released from the gas generating portion 90 comes into contact with the measurement surface 82 of the concentration measuring portion 80. That is, the gas generating portion 90 is arranged at a position where the gas generated by the vaporizing portion 91 being driven floats upward in the medicinal solution 100 and comes into contact with the measurement surface 82.

In the present embodiment, the vaporizing portion 91 of the gas generating portion 90 is arranged right under the measurement surface 82 of the concentration measuring portion 80 in the medicinal solution tank 20, and gas generated by the vaporizing portion 91 floats upward in the medicinal solution 100 and comes into contact with the measurement surface 82, as an example. Note that, in a case where the gas generating portion 90 has a conduit or the like configured to guide the gas generated by the vaporizing portion 91 to the measurement surface 82, the vaporizing portion 91 may be arranged at a position not right under the measurement surface 82.

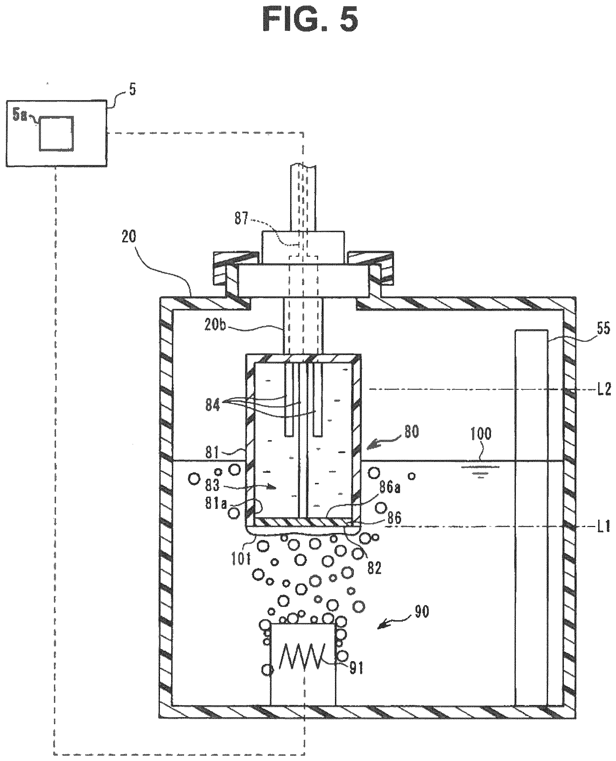

By driving the gas generating portion 90 and continuously releasing gas for a predetermined time period or longer in a state that the measurement surface 82 is submerged in the medicinal solution 100 in the medicinal solution tank 20, the measurement surface 82 is covered with gas 101 and is in a state of being in contact with the gas 101 as shown in Fig. 5.

Further, by stopping the gas generating portion 90 from being driven, after the measurement surface 82 is covered with the gas 101 generated by the gas generating portion 90 being driven, the gas 101 flows away from right under the measurement surface 82, and, therefore, the measurement surface 82 enters the state of being in contact with the medicinal solution 100.

Thus, by controlling a gas release operation by the gas generating portion 90 in the state that the measurement surface 82 of the permeation membrane 86 of the concentration measuring portion 80 is submerged in the medicinal solution 100, the endoscope reprocessor 1 of the present embodiment can switch between a first state that the measurement surface 82 is in contact with the medicinal solution 100 and a second state that the measurement surface 82 is in contact with the gas 101 generated by vaporization of the medicinal solution 100. That is, the controlling portion 5 performs control to create the first state by stopping the gas generating portion 90 and create the second state by driving the gas generating portion 90.

Next, an operation of the endoscope reprocessor 1 will be described.

When it is detected by the water level sensor 55 that the medicinal solution 100 is stored up to the first water level L1 or higher in the medicinal solution tank 20, the controlling portion 5 executes concentration measurement of the medicinal solution 100 by the concentration measuring portion 80. That is, a concentration measurement operation is executed if the measurement surface 82 of the permeation membrane 86 of the concentration measuring portion 80 is submerged in the medicinal solution 100. Further, when the concentration measurement operation is started, the gas generating portion 90 is in a stop state.

Fig. 4 shows a flowchart of the concentration measurement operation of the endoscope reprocessor 1. As shown in Fig. 4, in the concentration measurement operation, the controlling portion 5 judges whether the permeation membrane 86 is in a dry state or not by the judging portion 5a at step S10 first. Here, the dry state refers to a case where the amount of moisture in the second area 86c of the section of the permeation membrane 86 is equal to or below a predetermined value or a case where the second area 86c does not exist on the section of the permeation membrane 86, and the dry area 86d reaches the measurement surface 82.

Further, "judgement" by the judging portion 5a stated here is a process performed based on a value of some variable on a program executed by the controlling portion 5, and is not limited to a form of directly recognizing the dry state of the permeation membrane 86 by a sensor or the like provided in the endoscope reprocessor 1. For example, at step S10, the controlling portion 5 may presume whether the permeation membrane 86 is in a dry state or not based on stored information about dates and time of operations of the endoscope reprocessor 1 in past and cause the presumption to be a judgement result. Further, for example, at step S10, the controlling portion 5 may judge whether the permeation membrane 86 is in a dry state or not based on information inputted to the operation portion 7 by the user.

In the present embodiment, in a case of a concentration measurement operation executed for the first time after an operation of diluting the medicinal solution 100 is executed in the medicinal solution tank 20, the controlling portion 5 judges that the permeation membrane 86 is in a dry state at step S10, as an example. The operation of diluting the medicinal solution 100 is an operation of mixing unused stock solution of the medicinal solution and water at a predetermined ratio in the medicinal solution tank 20 after discharging used medicinal solution 100 in the endoscope reprocessor 1 outside the apparatus.

Note that a process of the controlling portion 5 judging that the permeation membrane 86 is in a dry state if a concentration measurement operation being executed is an operation executed for the first time after the measurement surface 82 is exposed continuously in gas for a predetermined time period or longer may be added to step S10. The period during which the measurement surface 82 is exposed in gas includes a period of a state that the used medicinal solution 100 in the endoscope reprocessor 1 has been discharged outside the apparatus. In this case, if it is detected by the water level sensor 55 that a state that the liquid surface in the medicinal solution tank 20 is lower than the first water level L1 has continued for a predetermined time period or longer, the controlling portion 5 judges that the permeation membrane 86 is in a dry state.

If it is judged at step S10 that the permeation membrane 86 is not in a dry state (step S20: NO), the flow proceeds to step S30. That is, if it is judged that the amount of moisture of the second area 86c of the permeation membrane 86 exceeds the predetermined value, and the measurement surface 82 is in a wet state, then the flow proceeds to step S30.

At step S30, the controlling portion 5 sets the first state that the gas generating portion 90 is stopped. Then, at step S40, the controlling portion 5 drives the concentration measuring portion 80 to execute concentration measurement.

Thus, if the permeation membrane 86 is not in a dry state, the controlling portion 5 of the present embodiment executes first control to drive the concentration measuring portion 80 in the state that the gas generating portion 90 is stopped.

In the first control, the measurement surface 82 is in contact with the medicinal solution 100, which is liquid, when step S40 is executed. Therefore, at step S40, the concentration measuring portion 80 directly measure a concentration of the medicinal solution 100.

On the other hand, if it is judged at step S10 that the permeation membrane 86 is in a dry state (step S20: YES), the flow proceeds to step S50. That is, if it is judged that the amount of moisture of the second area 86c of the permeation membrane 86 is equal to or below the predetermined value, the flow proceeds to step S50.

At step S50, the controlling portion 5 sets the second state that the gas generating portion 90 is driven. Then, at step S60, the controlling portion 5 drives the concentration measuring portion 80 to execute concentration measurement. After completion of step S60, the controlling portion 5 stops the gas generating portion 90 at step S70.

Thus, if the permeation membrane 86 is in a dry state, the controlling portion 5 of the present embodiment executes second control to drive the concentration measuring portion 80 in the state that the gas generating portion 90 is driven. Note that, in the second control, a standby operation of waiting for a predetermined time period until the measurement surface 82 is covered with the gas 101 generated by the gas generating portion 90 being driven may be provided before execution of step S60.

In the second control, the measurement surface 82 is in contact with the gas 101 generated by vaporization of the medicinal solution 100 when step S60 is executed. Since the gas 101 is obtained by vaporizing the medicinal solution 100 stored in the medicinal solution tank 20, there is a correlation such as a proportional relation between a concentration of a measurement target component in the gas 101 and the concentration of the measurement target matter in the medicinal solution 100 stored in the medicinal solution tank 20. Therefore, at step S60, the concentration measuring portion 80 can perform concentration measurement of the medicinal solution 100 by measuring the concentration of the gas 101.

As described above, in the concentration measurement operation, if the measurement surface 82 of the permeation membrane 86 of the concentration measuring portion 80 is in a wet state, the endoscope reprocessor 1 of the present embodiment executes the second control to cause the medicinal solution 100 to be in contact with the measurement surface 82 to measure the concentration of the medicinal solution 100. If the measurement surface 82 is in a wet state, the concentration of the measurement target matter in the internal liquid 83 instantly changes according to the concentration of the measurement target matter in the medicinal solution 100 in contact with the measurement surface 82, and, therefore, it is possible to execute concentration measurement of the medicinal solution 100 by the concentration measuring portion 80 without delay.

Further, in the concentration measurement operation, if the measurement surface 82 of the permeation membrane 86 of the concentration measuring portion 80 is in a dry state, the endoscope reprocessor 1 of the present embodiment executes the second control to cause the gas 101 generated by vaporization of the medicinal solution 100 to be in contact with the measurement surface 82 to measure the concentration of the medicinal solution 100. If the measurement surface 82 is in a dry state, the measurement target component in the gas 101 in contact with the measurement surface 82 instantly permeates the dry area 86d of the permeation membrane 86 and reaches the inside of the internal liquid 83, and therefore, it is possible to execute concentration measurement of the medicinal solution 100 by the concentration measuring portion 80 without delay.

Thus, the endoscope reprocessor 1 of the present embodiment can retain an amount of permeation of the measurement target component through the permeation membrane 86 per unit time period at a predetermined value or above irrespective of the dry state of the measurement surface 82, by switching fluid to be in contact with the measurement surface 82 between liquid and gas according to the dry state of the measurement surface 82 of the permeation membrane 86 of the concentration measuring portion 80. In other words, in the present embodiment, it is possible to cause a rate of response of the concentration of the measurement target matter in the internal liquid 83, which changes according to the concentration of the measurement target matter in the medicinal solution 100, to be a predetermined value or above.

As described above, the endoscope reprocessor 1 of the present embodiment can execute concentration measurement of the medicinal solution 100 by the concentration measuring portion 80 without delay even if the permeation membrane 86 of the concentration measuring portion 80 is in a dry state.

Therefore, even in a case where the medicinal solution 100 does not exist in the medicinal solution tank 20 for a long period, and the permeation membrane 86 is in a dry state, for example, like a case of discharging the medicinal solution 100 from inside the medicinal solution tank 20 on a weekend and supplying new unused medicinal solution into the medicinal solution tank 20 on a workday, the endoscope reprocessor 1 of the present embodiment can execute concentration measurement of the medicinal solution 100 by the concentration measuring portion 80 without delay after supplying the medicinal solution 100 into the medicinal solution tank 20 and start succeeding reprocessing.

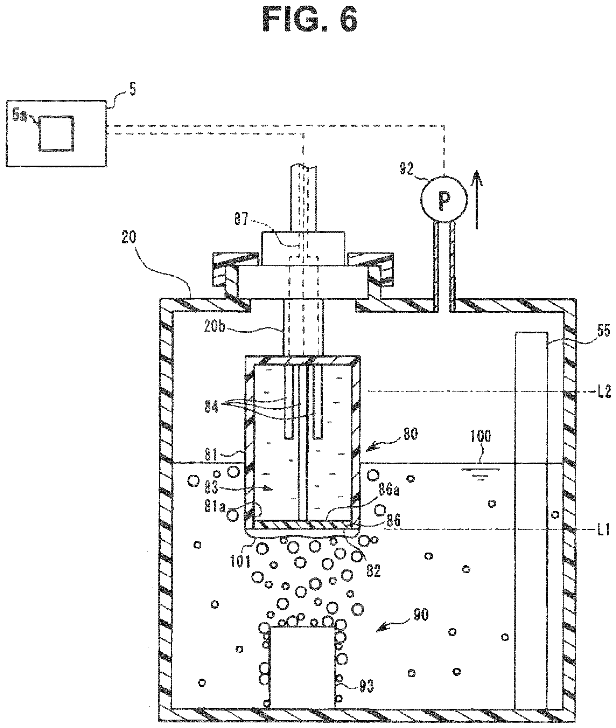

Note that, though the vaporizing portion 91 is configured being provided with a heater in the present embodiment described before, the configuration of the vaporizing portion 91 is not limited to that of the present embodiment. Fig. 6 shows a modification of the vaporizing portion 91.

The vaporizing portion 91 of the modification shown in Fig. 6 is provided with a vacuum pump 92 and a vaporization promoting portion 93. The vacuum pump 92 discharges air in the medicinal solution tank 20 by being driven, and causes the pressure of the medicinal solution 100 to be lower than the saturated vapor pressure.

The vaporization promoting portion 93 is a member with fine projections and recesses formed on a surface or a porous member, and promotes occurrence of nucleation of the medicinal solution 100. The vaporization promoting portion 93 is arranged right under the measurement surface 82 of the concentration measuring portion 80.

In the present modification, if the pressure of the medicinal solution 100 becomes lower than the saturated vapor pressure by the vacuum pump 92 being driven, air bubbles are generated in the entire medicinal solution 100. Here, since an amount of generation of air bubbles on the surface of the vaporization promoting portion 93 submerged in the medicinal solution 100 is larger than other places, it is possible to shorten a time period required to cover the measurement surface 82 with the gas 101.

### (Second Embodiment)

Next, a second embodiment of the present invention will be described. Only points different from the first embodiment will be described below. Components similar to those of the first embodiment will be given same reference numerals, and description of the components will be appropriately omitted.

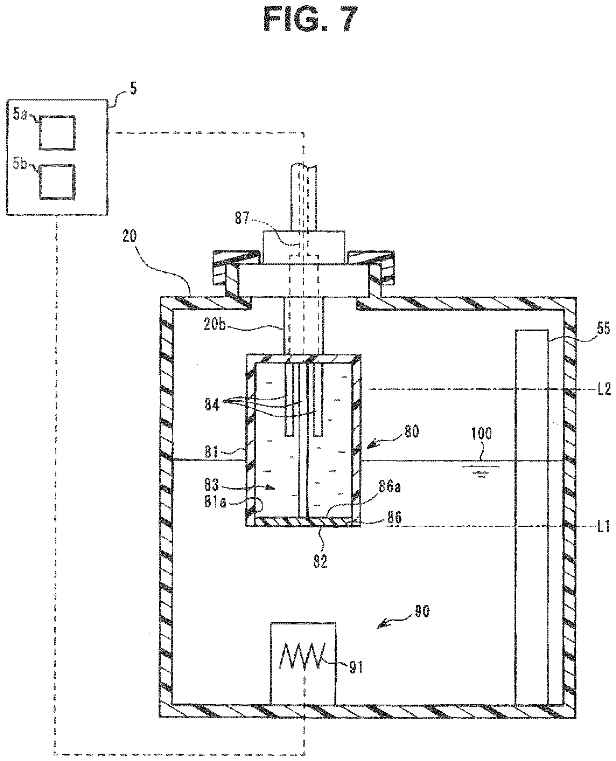

In the endoscope reprocessor 1 of the present embodiment, the controlling portion 5 is provided with a concentration judging portion 5b as shown in Fig. 7. The concentration judging portion 5b executes a concentration judgement operation of comparing a measurement result of the concentration measuring portion 80 with a comparison criterion to judge whether the concentration of the medicinal solution 100 has reached a reference value or not.

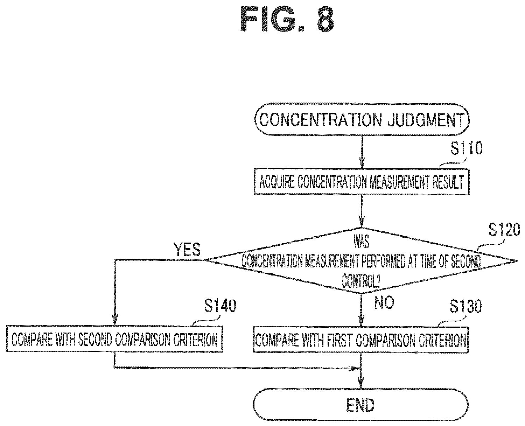

Fig. 8 is a flowchart of the concentration judgement operation. The concentration judgement operation is performed after the concentration measurement operation in Fig. 4 described in the first embodiment is completed.

In the concentration judgement operation, the concentration judging portion 5b reads a measurement result of the concentration measuring portion 80 by the concentration measurement operation at step S110 first. Next, at step S120, the concentration judging portion 5b judges whether concentration measurement performed to obtain the measurement result read at step S110 was performed at the time of the second control.

The second control is such control that, after driving the gas generating portion 90 to cause the gas 101 obtained by vaporizing the medicinal solution 100 to be in contact with the measurement surface 82 of the concentration measuring portion 80, the controlling portion 5 drives the concentration measuring portion 80 as described in the first embodiment. That is, at step S120, it is judged whether or not the measurement result was obtained by driving the concentration measuring portion 80 in the state that the gas 101 obtained by vaporizing the medicinal solution 100 is in contact with the measurement surface 82.

If it is judged at the judgement of step S120 that the concentration measurement was not performed at the time of the second control but was performed at the time of the first control, the flow proceeds to step S130. Note that the first control is such control that the controlling portion 5 drives the concentration measuring portion 80 in the state that the gas generating portion 90 is stopped, as described in the first embodiment, and the measurement surface 82 is in contact with the medicinal solution 100 at the time of driving the concentration measuring portion 80.

At step S130, the concentration judging portion 5b compares the measurement result read at step S110 and a predetermined first comparison criterion. Here, the first comparison criterion is a lower limit of a concentration at which the medicinal solution 100 exerts efficacy required for reprocessing.

If the measurement result read at step S110 is equal to or above the first comparison criterion as a result of the comparison at step S130, the concentration judging portion 5b judges that the concentration of the medicinal solution 100 stored in the medicinal solution tank 20 is such a value that the medicinal solution 100 exerts the efficacy required for reprocessing.

On the other hand, if it is judged at the judgement of step S120 that the concentration measurement was performed at the time of the second control, the flow proceeds to step S140.

At step S140, the concentration judging portion 5b compares the measurement result read at step S110 and a predetermined second comparison criterion. Here, the second comparison criterion is a concentration of a measurement target component in gas obtained when the medicinal solution 100 with a lower-limit concentration at which the medicinal solution 100 exerts the efficacy required for reprocessing is vaporized.

If the measurement result read at step S110 is equal to or above the second comparison criterion as a result of the comparison at step S140, the concentration judging portion 5b judges that the concentration of the medicinal solution 100 stored in the medicinal solution tank 20 is such a value that the medicinal solution 100 exerts the efficacy required for reprocessing.

As described above, the endoscope reprocessor 1 of the present embodiment uses the first comparison criterion and the second comparison criterion, which are different comparison criteria, in the case of judging whether the medicinal solution 100 is efficacious or not based on a measurement result obtained by driving the concentration measuring portion 80 in the state that the medicinal solution 100 is in contact with the measurement surface 82 (at the time of the first control) and in the case of judging whether the medicinal solution 100 is efficacious or not based on a measurement result obtained by driving the concentration measuring portion 80 in the state that the gas 101 obtained by vaporizing the medicinal solution 100 is in contact with the measurement surface 82 (at the time of the second control) by the concentration judging portion 5b.

Thereby, it is possible to eliminate deviation between judgements about whether the medicinal solution 100 is efficacious or not at the time of the second control and at the time of the first control.

### (Third Embodiment)

Next, a third embodiment of the present invention will be described. Only points different from the first and second embodiments will be described below. Components similar to those of the first and second embodiments will be given same reference numerals, and description of the components will be appropriately omitted.

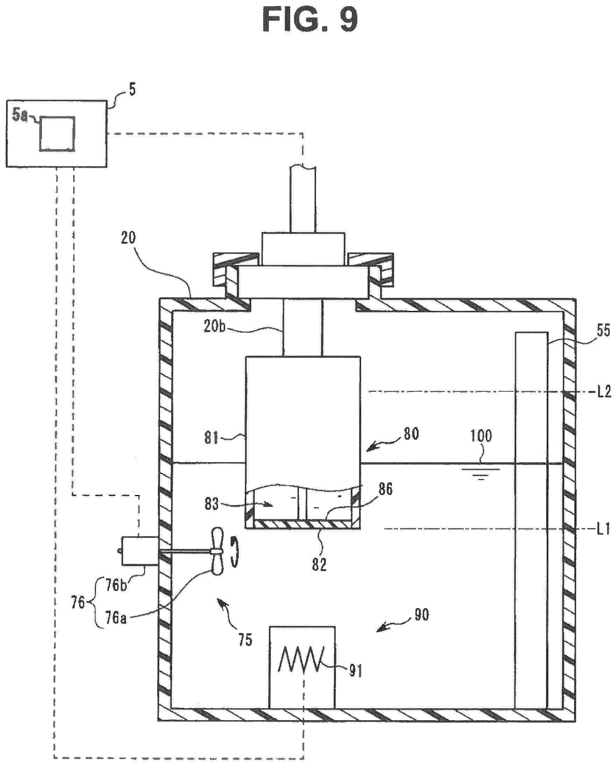

As shown in Fig. 9, the endoscope reprocessor 1 of the present embodiment is provided with a removing portion 75 configured to remove the gas 101 in contact with the measurement surface 82 of the permeation membrane 86 of the concentration measuring portion 80 by execution of the second control. The removing portion 75 is connected to the controlling portion 5, and operation of the removing portion 75 is controlled by the controlling portion 5.

The removing portion 75 is only required to have a configuration for promoting separation of the gas 101 from the measurement surface 82 in the medicinal solution 100, and the configuration is not especially limited. In the present embodiment shown in Fig. 9, the removing portion 75 is provided with a liquid flow generating portion 76 configured to cause a flow of the medicinal solution 100 to occur in a vicinity of the measurement surface 82, as an example.

The liquid flow generating portion 76 is provided with a vane 76a which is rotatably arranged, and an electric motor 76b configured to drive the vane 76a. When the electric motor 76b is driven in the case where the medicinal solution 100 is stored up to the first water level L1 of the medicinal solution tank 20 or higher, the medicinal solution 100 in the vicinity of the measurement surface 82 can be caused to flow by rotation of the vane 76a. Note that the liquid flow generating portion 76 may also have a configuration for stirring the medicinal solution 100 in the medicinal solution tank 20.

The controlling portion 5 executes the operation of removing the gas 101 which is in contact with the measurement surface 82 by the removing portion 75 after completion of step S70 shown in Fig. 4. By quickly removing the gas 101 in contact with the measurement surface 82 by the removing portion 75 after execution of concentration measurement, it is possible to cause the medicinal solution 100 to quickly permeate the measurement surface 82 of the permeation membrane 86.

Note that the configuration of the liquid flow generating portion 76 is not limited to that of the present embodiment. For example, the liquid flow generating portion 76 may be in a form of being provided with a pump configured to eject the medicinal solution 100 and causing the medicinal solution 100 in the vicinity of the measurement surface 82 to flow by the pump being driven.

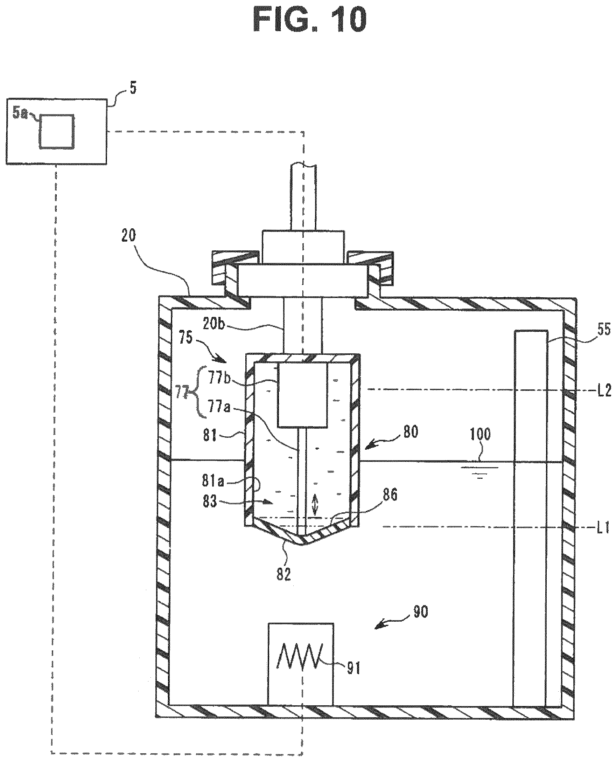

Fig. 10 shows a modification of the removing portion 75. The removing portion 75 of the present embodiment described before has the configuration in which air bubbles of the gas 101 in contact with the measurement surface 82 are removed by causing the medicinal solution 100 in the vicinity of the measurement surface 82 to flow. On the other hand, the removing portion 75 of the present modification has a configuration in which, by forming an inclined plane which forms a predetermined angle relative to a horizontal plane, on the measurement surface 82, the gas 101 in contact with the measurement surface 82 is caused to move by buoyancy and removed.

As shown in Fig. 10, the removing portion 75 of the present modification is provided with a slope forming portion 77 configured to give a slope to the measurement surface 82. As an example, the slope forming portion 77 is provided with a piston 77a configured to press a central part of the permeation membrane 86 from inside the recess 81a of the housing 81, and an actuator 77b configured to drive the piston 77a.

By pressing the central part of the permeation membrane 86 by the piston 77a, the permeation membrane 86 is deformed so as to project to an outer side of the housing 81, and the measurement surface 82 becomes an inclined plane as shown in Fig. 10. Thereby, the gas 101 in contact with the measurement surface 82 moves upward by buoyancy and leaves the measurement surface 82.

Note that the configuration of the slope forming portion 77 is not limited to the configuration shown in Fig. 10. For example, the slope forming portion 77 may be in a form of deforming the measurement surface 82 so as to project to the outer side of the measurement surface 82 by increasing pressure in the recess 81a of the housing 81 by a pump or the like. Further, for example, the slope forming portion 77 may be in a form of deforming the measurement surface 82 so as to project to the outer side of the measurement surface 82 by pulling the central part of the permeation membrane 86 from outside the housing 81.

Further, for example, the slope forming portion 77 may be provided with an actuator configured to incline the housing 81 or the medicinal solution tank 20 and have a configuration in which, by inclining the housing 81 or the medicinal solution tank 20 including the housing 81, the measurement surface 82 is caused to be an inclined plane forming a predetermined angle relative to the horizontal plane.

Note that the endoscope reprocessor 1 of the present embodiment may be provided with the concentration judging portion 5b described in the second embodiment. By using the first comparison criterion and the second comparison criterion, which are different comparison criteria, in the case of judging whether the medicinal solution 100 is efficacious or not based on a measurement result obtained by execution of the first control and in the case of judging whether the medicinal solution 100 is efficacious or not based on a measurement result obtained by execution of the second control by the concentration judging portion 5b, it is possible to eliminate deviation between judgements about whether the medicinal solution 100 is efficacious or not at the time of the second control and at the time of the first control.

### (Fourth Embodiment)

Next, a fourth embodiment of the present invention will be described. Only points different from the first to third embodiments will be described below. Components similar to those of the first to third embodiments will be given same reference numerals, and description of the components will be appropriately omitted.

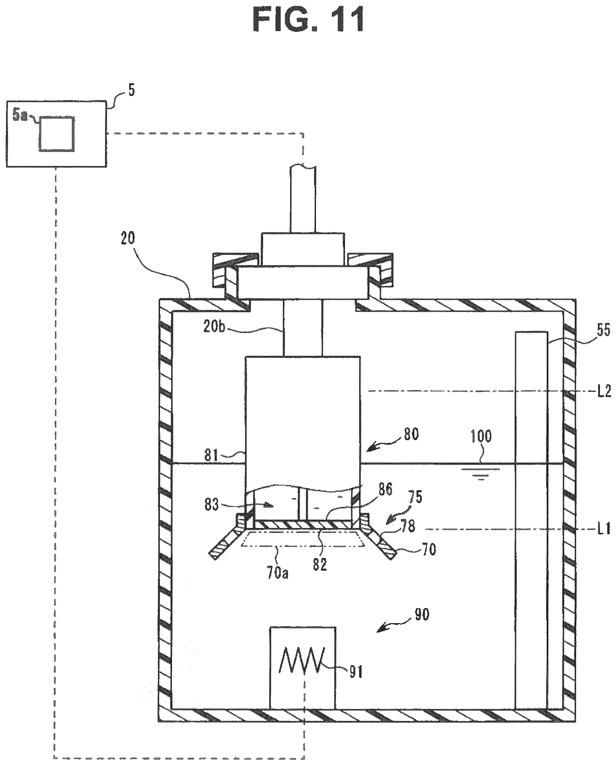

The endoscope reprocessor 1 of the present embodiment is provided with a collecting portion 70 configured to form a collection space 70a where the gas 101 which has floated upward in the medicinal solution 100 stored in the medicinal solution tank 20 is collected, as shown in Fig. 11. In the collection space 70a, the measurement surface 82 of the permeation membrane 86 of the concentration measuring portion 80 is arranged.

The collecting portion 70 is only required to be in a concave shape which opens downward so that the gas 101 which has floated upward in the medicinal solution 100 is stored. In the present embodiment shown in Fig. 11, the collecting portion 70 is such an umbrella-shaped member that an area of an opening increases downward, and a top part at a highest position on an inner side of the collecting portion 70 is the collection space 70a, as an example. The measurement surface 82 is arranged on the top part in the umbrella-shaped collecting portion 70.

The gas 101 which has floated upward into the umbrella-shaped collecting portion 70 is collected to the vicinity of the measurement surface 82 arranged on the top part and comes into contact with the measurement surface 82.

Thus, by being provided with the collecting portion 70, the endoscope reprocessor 1 of the present embodiment can retain the gas 101 generated by driving the gas generating portion 90 at the time of execution of the second control, at a position where the gas 101 is in contact with the measurement surface 82 and certainly cover the measurement surface 82 with the gas 101.

Further, since the area of the opening of the collecting portion 70 is larger than an area of the measurement surface 82, it is possible to collect the gas 101 floating upward in the medicinal solution 100 more and shorten a time period required to cover the measurement surface 82 with the gas 101.

Further, the endoscope reprocessor 1 of the present embodiment is provided with the removing portion 75 configured to remove the gas 101 in contact with the measurement surface 82 of the permeation membrane 86 of the concentration measuring portion 80. In the present embodiment, the removing portion 75 is configured with a hole 78 passing through the collecting portion 70, as an example. The hole 78 causes the collection space 70a and a space above the collection space 70a outside the collecting portion 70 to communicate with each other.

If the gas generating portion 90 is in the stop state, the gas 101 which has been collected in the collection space 70a of the collecting portion 70 and is in contact with the measurement surface 82 moves outside the collecting portion 70 through the hole 78. By the gas 101 moving outside the collecting portion 70 through the hole 78, the gas 101 in contact with the measurement surface 82 is removed. Thus, the removing portion 75 of the present embodiment does not have a movable portion and is simply configured.

Note that the removing portion 75 of the present embodiment may be configured to have a movable portion as described in the third embodiment. For example, the removing portion 75 of the present embodiment may be provided with the liquid flow generating portion 76 configured to cause the gas 101 which has been collected in the collection space 70a and is in contact with the measurement surface 82 to flow outside the collecting portion 75 by generating a flow of the medicinal solution 100 in the vicinity of the measurement surface 82 as shown in Fig. 9.

Note that the endoscope reprocessor 1 of the present embodiment may be provided with the concentration judging portion 5b described in the second embodiment. By using the first comparison criterion and the second comparison criterion, which are different comparison criteria, in the case of judging whether the medicinal solution 100 is efficacious or not based on a measurement result obtained by execution of the first control and in the case of judging whether the medicinal solution 100 is efficacious or not based on a measurement result obtained by execution of the second control by the concentration judging portion 5b, it is possible to eliminate deviation between judgements about whether the medicinal solution 100 is efficacious or not at the time of the second control and at the time of the first control.

### (Fifth Embodiment)

Next, a fifth embodiment of the present invention will be described. Only points different from the first to fourth embodiments will be described below. Components similar to those of the first to fourth embodiments will be given same reference numerals, and description of the components will be appropriately omitted.

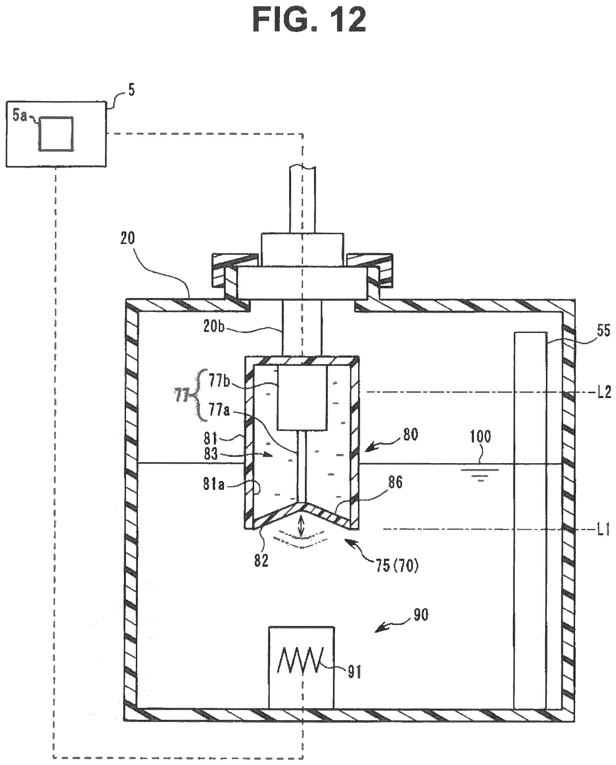

The endoscope reprocessor 1 of the present embodiment is provided with the collecting portion 70 configured to form the collection space 70a where the gas 101 which has floated upward in the medicinal solution 100 stored in the medicinal solution tank 20 is collected, and the removing portion 75 configured to remove the gas 101 in contact with the measurement surface 82 of the permeation membrane 86 of the concentration measuring portion 80 by execution of the second control, as shown in Fig. 12. In the present embodiment, the collecting portion 70 and the removing portion 75 are integrally configured unlike the fourth embodiment.

The collecting portion 70 includes the slope forming portion 77 and the permeation membrane 86. The slope forming portion 77 deforms the permeation membrane 86 into a concave shape recessed toward an inside of the recess 81 a of the housing 81 or a projection shape projecting toward the outer side of the housing 81. The slope forming portion 77 is connected to the controlling portion 5, and an operation of the slope forming portion 77 is controlled by the controlling portion 5.

As an example, the slope forming portion 77 is provided with the piston 77a configured to press and pull the central part of the permeation membrane 86 from inside the recess 81 a of the housing 81, and the actuator 77b configured to drive the piston 77a.

By pulling the central part of the permeation membrane 86 toward the inner side of the recess 81a by the piston 77a, the permeation membrane 86 becomes a concave shape recessed toward the inside of the recess 81a. In this state, the measurement surface 82 of the permeation membrane 86 becomes the collecting portion 70 in a concave shape which opens downward.

By being provided with the collecting portion 70, the endoscope reprocessor 1 of the present embodiment can retain the gas 101 generated by driving the gas generating portion 90 at the time of execution of the second control, at a position where the gas 101 is in contact with the measurement surface 82 and certainly cover the measurement surface 82 with the gas 101.

Further, by pressing the central part of the permeation membrane 86 toward an outer side of the recess 81a by the piston 77a, the permeation membrane 86 becomes a projection shape projecting to the outer side of the housing 81 (a state shown by two-dot chain lines in Fig. 12). In this state, since an inclined plane forming a predetermined angle relative to the horizontal plane is formed on the measurement surface 82, the measurement surface 82 of the permeation membrane 86 becomes the removing portion 75 configured to cause the gas 101 in contact with the measurement surface 82 to move by buoyancy to remove the gas 101.

Since the endoscope reprocessor 1 of the present embodiment is provided with the removing portion 75, the endoscope reprocessor 1 can cause the medicinal solution 100 to quickly permeate the measurement surface 82 of the permeation membrane 86, by quickly removing the gas 101 in contact with the measurement surface 82 by the removing portion 75 after execution of concentration measurement.

As described above, by configuring the collecting portion 70 and the removing portion 75 by a same member, the endoscope reprocessor 1 of the present embodiment can be simply configured.

Note that the endoscope reprocessor 1 of the present embodiment may be provided with the concentration judging portion 5b described in the second embodiment. By using the first comparison criterion and the second comparison criterion, which are different comparison criteria, in the case of judging whether the medicinal solution 100 is efficacious or not based on a measurement result obtained by execution of the first control and in the case of judging whether the medicinal solution 100 is efficacious or not based on a measurement result obtained by execution of the second control by the concentration judging portion 5b, it is possible to eliminate deviation between judgements about whether the medicinal solution 100 is efficacious or not at the time of the second control and at the time of the first control.

Note that the present invention are not limited to the embodiments described before but can be appropriately changed within a range not departing from the spirit or idea of the present invention read from Claims and the whole specification. Endoscope reprocessors in which such a change has been made are also included within the technical scope of the present invention.

According to the present invention, it is possible to realize an endoscope reprocessor capable of executing concentration measurement without delay even if a permeation membrane of a concentration measuring portion is in a dry state.

The present application is filed claiming priority based on Japanese Patent Application No. 2015-144145 filed in Japan on July 21, 2015, the disclosed content of which is incorporated in the specification and claims of the present application by reference.

## Claims

1. An endoscope reprocessor comprising:
a medicinal solution tank configured to store medicinal solution;
a concentration measuring portion arranged in the medicinal solution tank so that a permeation membrane is submerged in the medicinal solution;
a gas generating portion comprising a vaporizing portion configured to vaporize a part of the medicinal solution to generate gas and arranged at a position where the gas that floats upward in the medicinal solution is in contact with the permeation membrane; and
a controlling portion connected to the concentration measuring portion and the gas generating portion and configured to perform first control to drive the concentration measuring portion in a state that the gas generating portion is stopped and second control to drive the concentration measuring portion in a state that the gas generating portion is driven.

2. The endoscope reprocessor according to claim 1, comprising a collecting portion configured to form a collection space where the gas that floats upward in the medicinal solution is collected; wherein
the permeation membrane is arranged in the collection space.

3. The endoscope reprocessor according to claim 1, comprising a removing portion configured to remove the gas in contact with the permeation membrane.

4. The endoscope reprocessor according to claim 1, wherein the vaporizing portion is a heater.

5. The endoscope reprocessor according to claim 4, comprising:
a temperature measuring portion configured to measure a temperature of the medicinal solution; and
an adjusting portion configured to adjust output of the vaporizing portion;
wherein
the controlling portion controls the adjusting portion in accordance with a measurement result of the temperature measuring portion.

6. The endoscope reprocessor according to claim 1, comprising a concentration judging portion configured to compare a measurement result of the concentration measuring portion and a comparison criterion to judge whether a concentration of the medicinal solution reaches a reference value; wherein
the comparison criterion includes a first comparison criterion applied at time of the first control and a second comparison criterion applied at time of the second control.

7. The endoscope reprocessor according to claim 1, wherein the medicinal solution tank is a disinfectant liquid tank configured to store disinfectant liquid.
